# EUROPEAN PATENT APPLICATION

(11) **EP 2 787 074 A1**
(43) Date of publication of application: **08.10.2014**
(21) Application number: 12853525.9
(22) Date of filing: 30.11.2012
(51) Int. Cl.: C12N 5/077, C12M 3/00

(54) **METHOD FOR LONG-TERM STORAGE OF POROUS BODY BEARING CHONDROCYTES**

(30) Priority: 01.12.2011 JP 2011263837
(71) Applicant: Fujisoft Incorporated, Yokohama-shi, Kanagawa 231-8008 (JP)
(72) Inventor: HARAI, Motohiro, Yokohama-shi Kanagawa 231-8008 (JP); TATEYAMA, Toshiaki, Yokohama-shi Kanagawa 231-8008 (JP)
(74) Representative: Fuchs Patentanwälte Partnerschaft mbB
(86) International application number: PCT/JP2012/081134
(87) International publication number: WO 2013/081122

(57) **Abstract**

Disclosed is a method for storing a cartilage-cell-adhering porous body in an airtight state over a long term of at shortest 14 days. The method comprises suspending isolated cartilage cells into atelocollagen at an administration cell concentration of 1.0 × 10⁷ to 1.0 × 10⁸ cells/ml to prepare a cell suspension, seeding the cell suspension to a biocompatible porous body, gelatinizing the cell suspension on the porous body to yield the cartilage-cell-adhering porous body, and keeping the cartilage-cell-adhering porous body at a temperature of 26 to 37°C in a medium having an amount of 1 mL or more per 1.0 × 10⁵ cells that are administrated living cells out of the cartilage cells, containing insulin, a fibroblast growth factor, and a 5% or more serum, while the porous body is shaken to set the shaking speed of the medium in the range of 0 to 2.8 cm/s.

## Description

### Technical Field

The present invention relates to a storage method for storing a porous body to which cartilage cells adhere over a long term.

### Background Art

Patent Literature 1 discloses a method of using an isotonic salt solution, such as a phosphoric acid buffer, a Ringer's solution type storage solution or an isotonic salt solution, such as physiologic saline solution, to store cultivated cartilage tissues obtained by cultivating cartilage cells at 2 to 25°C over 10 days.

Patent Literature 2 discloses a cell storage method of immersing cells in a cell storage solution, in which the rotation speed of adjacent two cell aggregates is measured, and on the basis of the rotation speed, the concentration or quantity of an energy source in the cell storage solution is varied to make the proliferating ability of the cells blunt while the activity of the cells is maintained.

The method described in Patent Literature 1 is intended for storage over a term (about 10 days) for the transportation thereof, and is not intended for a storage over a term of 14 days, which is desired in sterility tests prescribed according to the Japanese Pharmacopoeia.

In Patent Literature 2, the method is a method of varying the concentration or quantity of an energy source in a cell storage solution to adjust the rotation speed of adjacent two cell aggregates to be in a range making the proliferating ability of the cells blunt while the cell activity is maintained. Thus, this method is not applicable to any situation that culture media cannot be exchanged with each other.

### Citation List

### Patent Literatures

Patent Literature 1: Jpn. Pat. Appln. KOKAI Publication No. 2005-95152
Patent Literature 2: Japanese Patent No. 4230750

### Summary of Invention

### Technical Problem

An object of the present invention is to provide a method for storing a porous body to which cartilage cells adhere over a long term without requiring any culture-medium exchange.

### Solution to Problem

The present invention to solve the above problem is a method for storing a cartilage-cell-adhering porous body in an airtight state over a long term of at shortest 14 days, the method comprising:
suspending isolated cartilage cells into atelocollagen at an administration cell concentration of 1.0 × 10⁷ to 1.0 × 10⁸ cells/ml to prepare a cell suspension;
seeding the cell suspension to a biocompatible porous body;
gelatinizing the cell suspension on the porous body to yield the cartilage-cell-adhering porous body; and
keeping the cartilage-cell-adhering porous body at a temperature of 26 to 37°C in a medium having an amount of 1 mL or more per 1.0 × 10⁵ cells that are administrated living cells out of the cartilage cells, the medium containing insulin, a fibroblast growth factor, and a 5% or more serum, while the porous body is shaken to set the shaking speed of the medium in the range of 0 to 2.8 cm/s.

### Advantageous Effects of Invention

The present invention makes it possible to store, over a long term, a porous body to which cartilage cells adhere without exchanging a medium therefor. Accordingly, the sterile state of the cartilage-cell-adhering porous body can be verified according to a sterile test. Moreover, the invention makes it possible to prevent an excessive proliferation of the cartilage cells adhering to the porous body while the porous body is stored over a long term. Furthermore, the number of dead cells is small after the long-term storage; thus, even after such a long-term storage, a cartilage-cell-adhering porous body in a state suitable for transplantation can be provided. Thus, the invention can provide a method for storing a cartilage-cell-adhering porous body without exchanging a medium therefor over a longer term than storage-terms according to the prior art, for example, over a term for a sterile test prescribed in Japanese Pharmacopoeia, for example, 14 days. Also in countries or districts where no law obligates the storage over 14 days, it is conceived to vary somewhat a period from the preparing of a cartilage-cell-adhering porous body to the supply of the porous body to an organization where the porous body are transplanted to a patient. However, in this period also, the cartilage cells can be safely stored in the state that the cells live over at least 14 day.

### Brief Description of Drawings

FIG. 1 is a scheme chart showing a process for preparing porous bodies to which auricular cartilage cells adhere.
FIG. 2 is a schematic view illustrating a situation that a cell suspension containing atelocollagen is introduced into a porous body.
FIG. 3 is a schematic view illustrating a situation that a cell-adhering porous body is stored in an airtight container.
FIG. 4 is a schematic view illustrating a situation that the cell-adhering porous body is stored over a long term.
FIG. 5 is a graph showing a variation between the cell survival rates of cells that are being stored, dependently on the temperature for the storage.
FIG. 6 is a graph showing that the cell survival rate is not varied between specific storage temperatures.

### Description of Embodiments

### (1) Outline of Preparing and Storage of Porous Body to which Cartilage cells Adhere

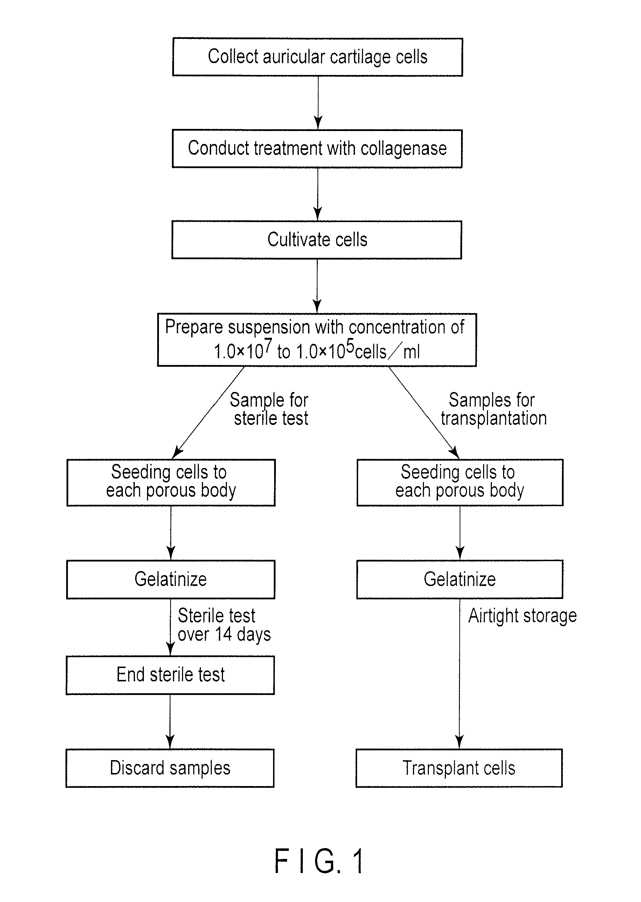

In the present specification, a "porous body to which cartilage cells adhere" may be referred to as a "cell-adhering porous body". A process of preparing cell-adhering porous bodies will be roughly described, referring to FIG. 1.

A cartilage is initially obtained from a subject. The cartilage obtained is subjected to an enzyme treatment, for example, collagenase treatment to isolate cartilage cells. Next, the isolated cells are suspended into a 2-3% atelocollagen solution to give a concentration of 1.0 × 10⁷ cells/mL to 1.0 × 10⁵ cells/mL. The resultant cell suspension is seeded to a porous body. Next, the resultant is allowed to stand still in an incubator of 37°C temperature for two hours to gelatinize atelocollagen, thereby fixing the cells onto the porous body. In this way, cell-adhering porous body is obtained. The cell-adhering porous body is transplanted into the subject and used therein. It is therefore necessary to examine whether or not the prepared cell-adhering porous body is sterile before the transplantation.

In the sterility test, for one rot, a plural number of cell-adhering porous bodies originating from the one cartilage are prepared and then a part of the cell-adhering porous bodies included in the one rot is/are subjected to sterility test. The cell-adhering porous body or bodies not subjected to the sterility test is/are airtightly stored in an airtight container, in parallel with the airtight storage of the cell-adhering porous body or bodies subjected to the sterilitytest. The airtight storage is maintained over at least the term for the sterility test. The airtight storage is performed by keeping the cell-adhering porous body in a preservation medium at a temperature of 26 to 37°C, while being shaken, the preservation medium having a volume of 1 mL or more per 1.0 × 10⁵ administered living cells, the preservation medium containing insulin, a fibroblast growth factor and a 5% or more serum, if the shaking is made linear, the shaking amplitude of the shaking is set into the range of 10 mm to 50 mm in at least one direction, if the shaking is made in a circular direction, the diameter of it is set into the range of 10 mm to 50 mm and the shaking speed set into the range of 15 to 90 rpm. After it is verified according to the sterility test that the rot is sterile, the airtightly stored cell-adhering porous body or bodies is/are used for transplantation.

### (2) Cell-Adhering Porous Body

The cell-adhering porous body contains a porous body and cartilage cells fixed on the surface of the porous body and inside surfaces of its pores.

### (3) Isolated Cells

The isolated cells are prepared as follows. Cartilage cells, for example, auricular cartilage cells are collected. Next, fats, blood vessels, blood and others are removed from the cartilage cells collected. The cartilage is made into thin strips. Furthermore, an enzyme, for example, a collagenase solution is used to digest the strips, so that the isolated cells are obtained.

Before the isolated cells are used to prepare the cell-adhering porous bodies, the isolated cells are seeded into a laboratory dish coated with gelatin or a flask coated with gelatin. This is digested with an enzyme such as trypsin, and the resultant is collected. In this way, the cells may be primarily cultivated, and continuously cultivated.

A growth medium for the primary cultivation and the continuously cultivation is a medium in which antibiotics such as penicillin and streptomycin are contained in a basal medium such as a DMEM/F12 or an MEM.

The step of fixing the isolated cells to the porous body to prepare the cell-adhering porous body is performed as follows: The isolated cells are suspended into an atelocollagen solution to yield a cell suspension. The atelocollagen solution contains atelocollagen, a basal medium, such as an MEM or a DMEM/F12, and antibiotics, such as penicillin and streptomycin.

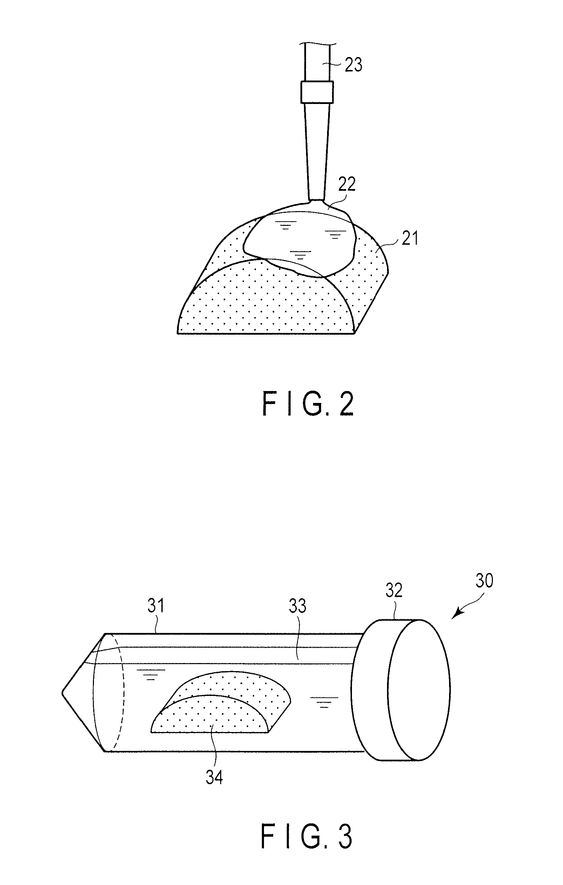

The cell suspension is added to the porous body that is, for example, semi-columnar porous body. As illustrated in FIG. 2, the addition of the cell suspension to the porous body may be performed by administrating the cell suspension that is a cell suspension 22 to the porous body that is a porous body 21 having a desired shape from the upper thereof by means of a pipette 23. The cell suspension administered to the porous body may contain the cells in a density of 1.0 × 10⁷ cells/mL to 1.0 × 10⁸ cells/mL.

The porous body may be made of a biocompatible porous material, for example, polylactic acid. The volume of the porous body may be decided, dependently on a site to which the porous body is to be transplanted. The porosity of the porous body is from 85 to 90%, and the average pore diameter thereof is about 400 µm.

The porous body to which the cells are administered is allowed to stand still in a sufficiently humid environment of 37°C temperature, for example, in the state of being put in a laboratory dish covered with lid in an incubator of 37°C temperature, and kept for about two hours. In this way, atelocollagen is gelatinized.

By the gelatinization of atelocollagen, the cell suspension is gelatinized, thereby the cells are fixed onto the surface of the porous body and inside surfaces of their pores, then the cell-adhering porous body is obtained.

The gelatinization of the atelocollagen solution may be performed, for example, as follows: a pedestal on which the porous body is to be put is set in a laboratory dish 9 cm in diameter in which 10 mL of a medium is put, the medium being, for example, a basal medium such as an MEM, a DMEM, a DMEM/F12 or a DMEM/F12 added with antibiotics such as penicillin and streptomycin; the porous body, into which the cells are administered, is put on the pedestal in the state that this cell-administered porous body does not sink into the medium so as to be made naked from the medium; and the cell-administered porous body is incubated in an incubator of, for example, 37°C for two hours in the state that the laboratory dish is lidded.

### (4) Airtight Storage

The airtight storage is illustrated in FIG. 3. A sterilized airtight container 30 is initially prepared. This airtight container 30 has: a one-side sealed cylinder 31 that has an open end region the outside surface of which is screw-cut; and a cap 32 that has an open end region the inside surface of which is screw-cut, and is to be screwed onto the screw of the one-side sealed cylinder 31. Next, a preservation medium 33 is held into the one-side sealed cylinder 31. Subsequently, any one 34 of the cell-adhering porous bodies is held into the one-side sealed cylinder 31. The cap 32 is screwed to the opening in the one-side sealed cylinder 31 to make the inside of the one-side sealed cylinder 31 airtight.

### (5) Shaking of Preservation Medium

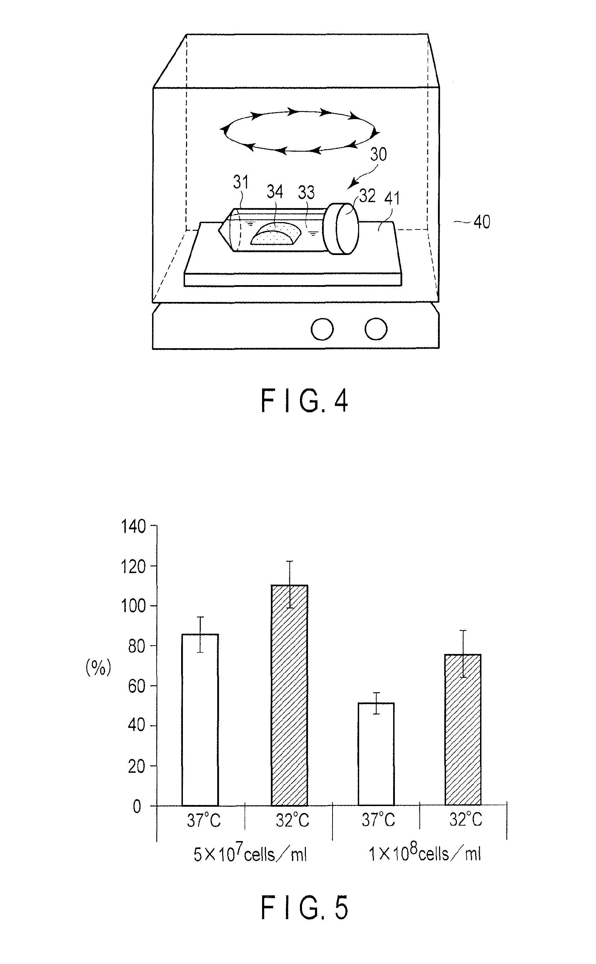

The shaking of the preservation medium 33 is described with reference to FIG. 4. Prepared is initially a temperature-constant shaker 40 in which a sliding pedestal 41 movable in, for example, an ellipsoid is set. The airtight container 30 is put onto the sliding pedestal 41 to make the axial direction of the container 30 substantially parallel to the top surface of the sliding pedestal 41. In the airtight container 30, the cell-adhering porous body 34 is held to cause the whole of the body 34 to sink in the preservation medium 33.

The container 30 is put onto the sliding pedestal 41, and then the inside of the temperature-constant shaker 40 is set into an environmental temperature of 26 to 37°C. The sliding pedestal 41 is then moved. By the movement of the sliding pedestal 41, the preservation medium 33 is shaken inside the container 30.

The movement of the sliding pedestal 41 may be the elliptical movement, or any movement except the elliptical movement, for example, circular movement, reciprocating movement, and/or figure-eight-shaped movement. By the movement of the sliding pedestal 41, the shaking amplitude of the preservation medium contained in the airtight container 30 is 10 to 50 mm in at least one direction when the shaking is made linear, or is 10 to 50 mm as a diameter at a shaking speed of 15 to 90 rpm when the shaking is performed into a circular direction. Such a shaking environment is achieved by an elliptical movement adjusted to set the rotating speed into the range of 15 to 90 rpm, using, for example, an ultra-compact temperature-constant shaking incubator (V·BR-36, manufactured by Taitec Co., Ltd.). The same shaking effect may be achieved by inclining, for example, the sliding pedestal 41 repeatedly within an inclination angle of, for example, 0 to 15°, using a shaker that is itself known.

When the preservation medium was shaken at an amplitude of 20 mm (according to a circular movement), the flow rate of the preservation medium 33 was measured with an instrument, Aplio (registered trademark) XG1202S, manufactured by Toshiba Corp. The results are as follows.

In the case of 15 rpm: no flow rate was measurable. However, it was able to be verified that the water surface of the preservation medium 33 in the airtight container 30 went up and down slightly, following the movement of the sliding pedestal 41. It therefore appears that a slight flow having a flow rate of 0 or more, which was unmeasurable with any meter, is created.

In the case of 30 rpm: no flow rate was measurable. However, it was able to be verified that in the same manner as in the case of 15 rpm, the water surface of the preservation medium 33 in the airtight container 30 went up and down slightly, following the movement of the sliding pedestal 41. It was observed that the up-and-down motion of the water surface was clearly larger in amplitude than in the case of 15 rpm. It therefore appears that a flow having a flow rate of 0 or more, which is unmeasurable with any meter, was created.

In the case of 60 rpm: the flow rate was from 0.5 to 1.0 cm/s. It was able to be verified that the water surface of the preservation medium 33 in the airtight container 30 went up and down clearly, following the movement of the sliding pedestal 41.

In the case of 90 rpm: the flow rate was from 1.6 to 2.8 cm/s. It was able to be verified the water surface of the preservation medium 33 in the airtight container 30 went up and down largely, following the movement of the sliding pedestal 41.

The shaking speed of the preservation medium may be greater than 0 cm/s and no greater than 2.8 cm/s, preferably U = a value from 1.6 cm/s to 2.8 cm/s. This speed is represented by the following expressions: when the flow rate of the preservation medium is represented by "u": 0 cm/s < u ≤ 2.8 cm/s, and U = a value from 1.6 cm/s to 2.8 cm/s.

In Examples described below, inspections are made under a condition that the amplitude of any shaker is 20 mm unless the amplitude is otherwise specified.

The preservation medium is a medium containing insulin, a fibroblast growth factor, and a 5%-or-more serum in a basal medium, for example, a DMEM/F12, and further containing antibiotics, such as penicillin and streptomycin.

According to the method of the present invention, a cell-adhering porous body can be stored over a long term, that is, over at shortest 14 days, without exchanging a medium therefor. This makes it possible to make a sterility test for verifying a sterile state of the cell-adhering porous body. This method also makes it possible to prevent excessive proliferation of the cell-adhering porous body over a long-term storage to make the number of dead cells out of the cells small after the long-term storage. Thus, the present invention can provide cell-adhering porous body in a state suitable for transplantation even after it is stored over a long term.

### [Examples]

### 1. Process for Preparing Cartilage-Cell-Adhering Porous Body

Each of cartilage-cell-adhering porous bodies used Examples 1 to 8, which will be described later, was prepared basically as described below.

### (1) Cartilage Tissue Isolation and Cell-Seeding (P0:Passage 0)

A cartilage tissue collected by a sterile operation was carried in CPC(Cell Processing Center), and then fats, blood and others were removed therefrom. Next, the resultant cartilage was cut into strips. The cartilage strips cut were put into a 1.2% collagen solution. An ultra-compact temperature-constant shaking incubator (V·BR-36, manufactured by Taitec Co., Ltd.) was used to shake the cartilage-put solution at 37°C at 120 rpm (according to a circular movement such that a circle having a major diameter of 20 mm was drawn; hereinafter, the shaking movement was the same unless otherwise specified) for 18 hours to isolate cells. A desired number of cells out of the isolated cells were suspended into a growth medium (a DMEM/F12 containing 5 µg/mL of insulin + 0.1 µg/mL of FGFs + a 5% serum + penicillin/streptomycin). The cell suspension was seeded into gelatin coated laboratory dishes at a concentration of 2 × 10⁵ cells/laboratory-dish. These were incubated at 37°C in a CO₂ incubator.

A 1.2% collagenase solution was prepared as follows. Collagen was weighed out in an amount of 0.3g, and the weighed-out collagen was deposited in a 50-mL tube. Thereto was added 25 mL of a DMEM/F12 to dissolve the collagen therein completely.

A culture medium was each prepared as follows. Into a medium bottle was added 450 mL of a DMEM/F12 by means of a 50-mL pipette. Next, the 5-mL pipette was used to add thereto 5 mL of penicillin/streptomycin of 100 units/mL penicillin + 0.1 mg/mL. Furthermore, thereto were added 500 µL of FGFs having a quantity of 100 µg/mL by means of a 1000µL chip. Furthermore, thereto was added 710 µL of NOVOLIN R as insulin by means of a 1000µL chip. Thereto was added 25 ml of serum by means of a 25-mL pipette, and then all of the components were sufficiently mixed with each other.

### <P0 Cell Medium Exchange>

About the cells in item (1), a medium exchange was made as follows. From the gelatin coated laboratory dishes containing the cells in item (1) that were being cultured, the each medium was removed by means of an aspirator. Next, 10 mL of a growth medium was added to each of the dishes. The resultants were incubated at 37°C in a CO₂ incubator. The medium exchange was carried out at intervals of three to four days.

### (2) Passage culture (P0:Passage 0 → P1:Passage 1)

Subculture was performed as follows. The cells were detached from the gelatin coated laboratory dishes containing the primary cultured cells by trypsin treatment, and then put together into a tube. The collected cells were suspended into a growth medium (a DMEM/F12 containing 5 µg/mL of insulin + 0.1 µg/mL of FGFs + a 5% serum + penicillin/streptomycin). The resultant cell suspension was seeded into 18 gelatin coated flasks at a concentration of 1.5 × 10⁵ cells/flask. These were incubated at 37°C in a CO₂ incubator.

### <Medium Exchange>

In the passage culture, a medium exchange was performed as follows. An aspirator was used to remove the media from the gelatin coated flasks. Next, 20L of a growth medium was added in each of the gelatin coated flasks. These were incubated at 37°C in a CO₂ incubator.

### (3) Cell Collection, and Cell Administration to Porous Bodies

The cells were detached from the 18 gelatin coated flasks, and then put together into a tube so as to be collected. The collected cell was separated from cell suspension by centrifugation, and then put into an MEM containing penicillin/streptomycin. This was partially used to determine the number of cells. On the basis of the cell count, a cell suspension having a volume corresponding to 2.25 × 10⁸ cells was transferred into a 25-mL tube, and centrifuged. The resultant supernatant was then removed. The resultant cell pellets were suspended into an MEM containing penicillin/streptomycin to give a final liquid volume of 3.0 mL. This was mixed with 1.5 mL of a 3% atelocollagen. The ratio of the cell suspension to the atelocollagen was 2:1. After the mixing, the final concentration of atelocollagen was 1%. A 1-mL syringe was used to collect a volume of 700 µL therefrom. This was administered into each porous body, in a substantially semi-columnar form (6 [in width W] × 50 [in length L] × 3 [in height H]), made of polylactic acid. The quantity of the atelocollagen-containing cell suspension to be added to the porous body was decided in accordance with the size of the porous body. For example, when the porous body had a size of 6 (W) × 5 (L) × 3 (H), the administration quantity was set to 70 µL, and when it had a size of 10 (W) × 5 (L) × 3 (H), a quantity of 120 to 150 µL was administered.

### (4) Fixation of Cells

Next, the porous bodies to which the cells were administered were each arranged in each of laboratory dishes in which a preservation medium containing penicillin/streptomycin was put, and the laboratory dishes were allowed to stand still at 37°C in an incubator for two hours to gelatinize atelocollagen. In this way, cell-adhering porous bodies were obtained.

### 2. Method for Measuring the Number of Cells

In each of Examples described below, the number of cells is measured, using an instrument NucleoCounter (registered trademark) manufactured by a company, Chemometec, Reagent A100 and Reagent B100 (manufactured by the company Chemometec) as cell treatment reagent, and an exclusive-use sample cassette NucleoCasette (registered trademark).

The concentration of entire cells therein is measured as described below.

### Preparation of Sample

Into a test tube is collected 50 mL of a suspension of cells to be measured, and thereto is added 50 µL of each of the cell treatment reagents Reagent A100 and Reagent B100 to prepare a sample.

### Measurement of Concentration of Entire Cells

In the measurement of the concentration of the entire cells, the sample prepared in the just-above-described item "Preparation of Sample" was drawn into the NucleoCasette (registered trademark), and this was set into the NucleoCounter (registered trademark). Next, the Run button of the NucleoCounter (registered trademark) was pressed to start measuring the number of entire cells. Thus, the measurement result was obtained.

### Measurement of Concentration of Dead Cells

The concentration of dead cells was made as follows. 100 µL of the cultured cells was taken into a tube, and was drawn into the NucleoCasette (registered trademark), and this was set into the NucleoCounter (registered trademark). Next, the Run button of the NucleoCounter (registered trademark) was pressed to start measuring the number of dead cells. Thus, the measurement result was obtained.

The cell survival rate was calculated, using the following calculating expressions
Living cell concentration = Entire cell concentration - Dead cell concentration; and
Cell survival rate = Living cell concentration / Entire cell concentration

### <Example 1>

### Composition of Preservation Medium

The composition of a preservation medium for cell-adhering porous bodies was decided. Using media each containing a combination of two or more out of, as a growth factor, insulin, FGFs (fibroblast growth factors), dexamethasone (for inducing the redifferentiation of cartilage cells), somatomedin (for inducing the redifferentiation of cartilage cells) and serum, effects on cells of cell-adhering porous bodies after a long-term storage thereof were investigated.

Each of the components contained in the media is represented by a symbol described below. In Table 1 described below is shown a correspondence between the component and the symbol, together with the use concentration thereof.

**Table 1**

| Abbreviations | | Used concentration |
|---|---|---|
| I | Insulin | 5 µg/ml |
| F | FGF: fibroblast growth factor | 0.1 µg/ml |
| D | Dexamethasone | 1 µM |
| C | Somatomedin C | 100 ng/ml |
| S | Serum | 5% |

### Experiment 1

Media A0, A1 and A2 were compared with each other. A DMEM was used as a basal medium for these media, which contained penicillin/streptomycin. The respective compositions of the media are as follows. Composition of medium A0: 5 µg/mL of insulin, 0.1 µg/mL of FGFs, and a 5% serum;
Composition of medium A1: 100 ng/mL of somatomedin,
5 µg/mL of insulin, 0.1 µg/mL of FGFs, and a 5% serum; and Composition of medium A2: 1 µM of dexamethasone,
5 µg/mL of insulin, 0.1 µg/mL of FGFs, and a 5% serum.

By comparing these media with each other, investigations were made about effects of dexamethasone and somatomedin, which are cartilage cell growth factors, on cells of cell-adhering porous body after a long-term storage thereof.

The cell-adhering porous bodies were prepared in accordance with the method described in items (1) to (4). Specifically, continuously-cultured cells prepared in accordance with items (1) and (2) in item 1, "Process for Preparing Cartilage-Cell-Adhering Porous Body," were used to prepare a cell suspension containing atelocollagen. The cell suspension having a cell concentration of 1.0 × 10⁷ cells/mL was administered to semi-columnar porous bodies each made of polylactic acid and having a size of 10 (W) × 5 (L) × 3 (H) so that the number of administrated living cells was adjusted to 1.5 × 10⁶ cells. These were allowed to stand still at 37°C in an incubator for two hours to gelatinize atelocollagen. Thereafter, 30 mL of medium A0, A1 or A2 was added onto 2% agarose pieces respectively and they were put respectively onto one thereof. The porous bodies were allowed to stand still on the pieces, respectively, and then maintained at an environmental temperature of 37°C in medium A0, A1 or A2 for two weeks.

Thereafter, the cell-adhering porous bodies were each treated with trypsin (TrypLE Express, manufactured by Gibco), and then cells were collected from the body. The numbers of entire cells, the numbers of living cells, and the cell-number ratios were calculated. Individual data are each shown as the average value from two experiment-examples.

The results are shown in Table 2.

**Table 2**

| | IFS | CIFS | DIFS |
|---|---|---|---|
| Number of administrated cells (cells) | 1.5×10⁶ | 1.5×10⁶ | 1.5×10⁶ |
| Survival rate (%) | 71.8 | 64.5 | 47.8 |
| Number of living cells (cells) | 4.0×10⁵ | 3.7×10⁵ | 4.3×10⁵ |
| Cell-number ratio (%) | 26.7 | 24.7 | 28.7 |

In the table, individual items show the following.

"Survival rate" represents the proportion of living cells in the entire cells after the storage for two weeks.

Dead cells include both of dead cells in which their membrane has been completely broken and dead cells in which their membrane has not been broken. When cells contain many dead cells, the former cells may unfavorably cause inflammation by leakage components from the cells after transplanted. It is therefore undesired that cells containing dead cells are used for transplantation.

"Cell-number ratio" is the ratio of the number of living cells recognized after the storage for two weeks to the number of administered cells before the two weeks.

"Number of living cells" is the number of living cells recognized after the storage for two weeks.

As shown in Table 2, in the cases of media A0, A1 and A2, the respective cell-number ratios were hardly different from each other. This matter has suggested that the storability of cartilage cells is not improved by the addition of dexamethasone and somatomedin. Accordingly, in experiments described below, the use of A0 media was decided, which contains 5 µg/mL of insulin, 0.1 µg/mL of FGFs and a 5% serum in a DMEM. In the case of any one of media A0, A1 and A2, the cell-number ratio was as low a vale as about 25%. This appears to be based on the storage by the still-standing. In the experiments described below, it was decided that shaking storage was to be performed.

### Experiment 2

In medium A0, it was checked whether or not FGFs and insulin produce an adverse effect onto cells of cell-adhering porous body when the body is stored over a long term. A comparison was made about respective effects of the following media onto such cells: medium A0 without FGFs, that is, a DMEM medium containing 5 µg/mL of insulin and a 5% serum (i.e., A3 medium); and medium A0 without FGFs and without insulin, that is, a DMEM medium containing a 5% serum (i.e., A4 medium).

Cell-adhering porous bodies were prepared in the same way as in Experiment 1. Continuously-cultured cells prepared in the above-mentioned items (1) and (2) were used to prepare a cell suspension containing atelocollagen. The cell suspension having a cell concentration of 1.0 × 10⁷ cells/mL was administered to semi-columnar porous bodies each made of polylactic acid and having a size of 10 (W) × 5 (L) × 3 (H) so that the number of administrated living cells was adjusted to 1.2 × 10⁶ cells. These were allowed to stand still at 37°C in an incubator for two hours to gelatinize atelocollagen. Thereafter, the resultants were each included into an airtight container in which 30 mL of medium A0, A3 or A4 was contained respectively. The containers were each stored for two weeks while being shaken at 37°C at a shaking speed of 15 rpm, using an ultra-compact temperature-constant shaking incubator (V·BR-36, manufactured by Taitec Co., Ltd.).

The compositions of the media are together described below.
Composition of medium A0: 5 µg/mL of insulin, 0.1 µg/mL of FGFs, and a 5% serum;
Composition of medium A3: 5 µg/mL of insulin, and a 5% serum; and
Composition of medium A4: a 5% serum.

The survival rates, the numbers of living cells, and the cell-number ratios were obtained. The results are shown in Table 3. The individual data are each shown as the average value from two experiment-examples.

**Table 3**

| | IFS | IS | S |
|---|---|---|---|
| Number of administrated cells (cells) | 1.2×10⁶ | 1.2×10⁶ | 1.2×10⁶ |
| Survival rate (%) | 94.9 | 84.8 | 85.9 |
| Number of living cells (cells) | 1.13×10⁶ | 3.08×10⁵ | 3.36×10⁵ |
| Cell-number ratio (%) | 88.9 | 24.2 | 26.3 |

From a comparison of medium A0 with media A3 and A4, it has been made evident that the cell-number ratio and the survival rate about medium A0 were each higher. It has also been made evident that shaking storage gave both of a higher cell-number ratio and a higher survival rate than standing-still storage. A reason therefor is presumed as follows: these storing media each contacted the cell-adhering porous body in the state of having some slight flow rate so that the preservation medium was easily supplied to the cells adhering to the porous body, or waste materials generated by the cells in the porous body flowed without staying at any one spot by flow of the preservation medium. In short, it is preferred that the flow rate of such a preservation medium is more than zero.

### Conclusion of Example 1

An object of the present invention is to use a cell-adhering porous body prepared by causing cells to adhere onto a porous body to store cartilage cells in a good state without exchanging a medium therefor over a period for a sterility test that is prescribed in Japanese Pharmacopoeia, that is, over 14 days. For this purpose, the survival rate and the cell-number ratio are each more preferably 100%. However, a matter that the cell-number ratio largely exceeds 100% means that the cells proliferate in the storage term. It is also feared that the excess cell proliferation is suspected canceration. It is therefore that the cell-number ratio after the storage period of two weeks is preferably about 100%. The cell-number ratio after the storage term according to the method of the present invention may be from about 80% to about 120%, preferably from about 90% to about 110%.

In Example 1, more preferred conditions for the survival rate and the cell-number ratio are conditions of Experiment 2 that medium A0 was used. Specifically, a more preferred survival rate and cell-number ratio were obtained in the case of using the DMEM containing 5 µg/mL of insulin, 0.1 µg/mL of the FGFs and the 5% serum as the preservation medium, and storing the cells under the shaking condition at 37°C. Accordingly, in experiments described below, it was decided that media A0 were to be used, and shaking storage were to be performed.

### <Example 2>

### Preservation Medium Amount and the Number of Administered Cells

Plural experiments were made to find out an optimal number of cartilage cells and an optimal amount of a medium for the purpose of storing the cells for 14 days in the state that the cells are seeded into a porous body.

### Experiment 3

Cell-adhering porous bodies were used to make an experiment to investigate the amount of a preservation medium necessary for storing the cell-adhering porous bodies satisfactorily.

A cell suspension containing atelocollagen was prepared in the same way as in the above-mentioned items (1) and (2). The cell suspension having an administration cell concentration of 1 × 10⁷ cells/mL was administered to semi-columnar porous bodies each made of polylactic acid and having a size of 10 (W) × 5 (L) × 3 (H) so that the number of administrated living cells was adjusted to 1.5 × 10⁶ cells. These were allowed to stand still at 37°C in an incubator for two hours to gelatinize atelocollagen. Then, cell-adhering porous bodies had been prepared. These were each put into a single-end sealed cylinder in which 30 mL or 300 mL of medium A0 was contained, and then the cylinder was capped. In this way, the cell-adhering porous bodies were each placed in the airtight container. This container was stored for two weeks while being shaken at an environmental temperature of 37°C at a shaking speed of 15 rpm, using an ultra-compact temperature-constant shaking incubator (V·BR-36, manufactured by Taitec Co., Ltd.).

Thereafter, the survival rates, the numbers of living cells, and the cell-number ratios were yielded. The results are shown in Table 4.

**Table 4**

| | | | |
|---|---|---|---|
| Administration cell concentration (cells/ml) | 1.0×10⁷ | | |
| Medium amount | | 30 ml | 300 ml |
| Day when measurement was made | Day 0 | Day 14 | |
| Survival rate (%) | 83.3 | 84.8 | 92.5 |
| Number of living cells (cells) | 4.2×10⁵ | 6.1×10⁵ | 1.9×10⁶ |
| Cell-number ratio (compared with that at day 0) | 100 | 145 | 450 |

When the cell suspension having an administration cell concentration of 1.0 × 10⁷ cells/mL to set the number of administrated cells to 1.5 × 10⁶ cells was used, the number of cells was increased in both the cases where the medium amount was 30 mL and 300 mL, respectively. This result has suggested that in the case of using a cell suspension having an administration cell concentration of 1 × 10⁷ cells/mL to set the number of administrated cells to 1.5 × 10⁶ cells, it is sufficient that the amount of a preservation medium is at least 30 mL.

### Experiment 4

The result of Experiment 3 has made it evident that it is sufficient that the amount of the preservation medium is 30 mL. Next, an investigation was made about the concentration of the administrated cells.

Cell-adhering porous bodies were stored for two weeks under the same conditions as in Experiment 3 except that only the administration cell concentration and the number of administrated cells were changed.

A cell suspension containing atelocollagen was prepared in the same way as in the above-mentioned items (1) and (2). The cell suspension having an administration cell concentration of 2 × 10⁷ cells/mL to porous bodies each made of polylactic acid and having a size of 10 (W) × 5 (L) × 3 (H) so that the number of administrated living cells was adjusted to 3 × 10⁶ cells. These were allowed to stand still at 37°C in an incubator for two hours to gelatinize atelocollagen. Then, cell-adhering porous bodies had been prepared. These were each put into a single-end sealed cylinder in which 30 mL of medium A0 was contained, and then the cylinder was capped. In this way, the cell-adhering porous body was placed in the airtight container. This was stored for two weeks while being shaken at an environmental temperature of 37°C at a shaking speed of 15 rpm, using an ultra-compact temperature-constant shaking incubator (V·BR-36, manufactured by Taitec Co., Ltd.).

Thereafter, the survival rate, the number of living cells, and the cell-number ratio were yielded. The results are shown in Table 5. The individual data are each shown as the average value from two experiment-examples.

**Table 5**

| | | |
|---|---|---|
| Administration cell concentration (cells/ml) | 2.0×10⁷ | |
| Medium amount | | 30 ml |
| Day when measurement was made | Day 0 | Day 14 |
| Survival rate (%) | 94.0 | 91.2 |
| Number of living cells (cells) | 1.55×10⁶ | 1.60×10⁶ |
| Cell-number ratio (compared with that at day 0) | 100 | 103 |

When the administration cell concentration was 2 × 10⁷ cells/mL and the number of administrated cells was 3 × 10⁶ cells, the cell-number ratio was 103% when the medium amount was 30 mL. This result has suggested that under the same conditions in Experiment 4, cells can be stored well.

### Experiment 5

As has been demonstrated in Experiment 4 described above, it was able to be verified that when the administration cell concentration was 2 × 10⁷ cells/mL and the number of administrated cells was 3 × 10⁶ cells, the cell-number ratio was 103% by using the medium in the amount of 30 mL. Next, an investigation was made about storage conditions, using porous bodies having a size permitting these bodies to be usable in actual regenerative medicine spots at a high probability.

Cell suspensions containing atelocollagen were prepared in the same way as in the above-mentioned items (1) and (2). The cell suspensions having an administration cell concentration of 5 × 10⁷ cells/mL or 1 × 10⁸ cells/mL was administered to a semi-columnar porous body made of polylactic acid and having a size of 6 (W) × 5 (L) × 3 (H) so that the number of administrated living cells was adjusted to 3.5 × 10⁶ cells or 7 × 10⁶ cells. Without gelatinizing atelocollagen therein, cell-adhering porous bodies were prepared. These were each put into a single-end sealed cylinder in which 50 mL of medium A0 was contained, and then the cylinder was capped. In this way, the cell-adhering porous bodies were each placed in the airtight container. This container was stored for two weeks while being shaken at an environmental temperature of 37°C at a shaking speed of 15 rpm, using an ultra-compact temperature-constant shaking incubator (V·BR-36, manufactured by Taitec Co., Ltd.).

The result of this experiment, Experiment 5, is herein compared with that of Experiment 4 described above. The administration cell concentrations in Experiment 5, which were 5.0 × 10⁷ cells/mL and 1.0 × 10⁸ cells/mL, were two times and five times the administration cell concentration in Experiment 4, respectively. However, the volume of the cell-adhering porous body used Experiment 5 was 60% of that of the cell-adhering porous body used in Experiment 4. Thus, when compared with Experiment 4 about the number of administrated cells relative to the volume of the porous body, Experiment 5 was approximately 1.17 times in the case of the administration cell concentration of 5.0 × 10⁷ cells/mL, and was approximately about 2.3 times in that of the administration cell concentration of 1.0 × 10⁸ cells/mL. Considering such conditions and the conditions in Experiment 2 for preparing good results, the preservation medium having an amount of 50 mL was used in Experiment 5.

After the cell-adhering porous bodies were stored under the above-mentioned conditions, the survival rate, the number of living cells, and the cell-number ratio were measured and calculated. The results are shown in Table 6. Data for day zero are shown as the average value from two experiment-examples thereof; and data for day 14, as the average value from three experiment-examples.

**Table 6**

| | | | | |
|---|---|---|---|---|
| Administration cell concentration (cells/ml) | 5.0×10⁷ | | 1.0×10⁸ | |
| Medium amount | 50 ml | | 50 ml | |
| Day when measurement was made | Day 0 | Day 14 | Day 0 | Day 14 |
| Survival rate (%) | 97.8 | 90.2 | 95.7 | 73.1 |
| Number of living cells (cells) | 2.74×10⁶ | 2.49×10⁶ | 5.97×10⁶ | 3.55×10⁶ |
| Cell-number ratio (compared with that at day 0) | 100 | 90.9 | 100 | 59.5 |

These results have made it evident that at the administration cell concentration of 5.0 × 10⁷ cells/mL, the cell-number ratio can be kept to be about 90% in a preservation medium amount of 50 mL. On the other hands, at the administration cell concentration of 1 × 10⁸ cells/mL, the cell-number ratio was 59.5%.

When the cell-adhering porous bodies were prepared at the administration cell concentration of 5 × 10⁷ cells/mL, the cell-number ratio was 90.9% after the storage for the two weeks by setting the amount of the preservation medium to 50 mL. Thus, it has been evaluated that under such conditions, the cell-adhering porous bodies can be satisfactorily stored.

It can be considered from these results that in order to store cartilage cells satisfactorily over a long term, it is important to adjust the amount of the preservation medium to satisfy appropriate conditions. However, the results have suggested that the content of nutrient components, such as serum, contained in the preservation medium may affect the storage. In other words, the results have suggested that when the amount of the preservation medium used relatively to the adopted number of administrated cells is small, the amount of nutrients supplied to the cells is small to result in a low cell-number ratio. This matter has suggested that by increasing the amount of the nutrient components contained in the preservation medium without increasing the amount of the preservation medium, that is, by increasing the amount of serum, the cells can be satisfactorily stored over a long term when the number of the administrated cells is any number.

The tendency of the redifferentiation of cartilage was inspected about cell-adhering porous bodies prepared at an administration cell concentration of 1 × 10⁸ cells/mL and cell-adhering porous bodies prepared at an administration cell concentration of 5 × 10⁷ cells/mL. Between the respective cases of these administration cell concentrations, no difference was generated in the tendency of the redifferentiation although experiment results thereof are not shown.

### <Example 3>

### Investigation on Shaking Speed

As has been demonstrated in Example 2 described above, the following has been made evident: several conditions for making it possible to produce satisfactory results, that is, the administration cell concentration, the amount of the preservation medium, storage environmental conditions and others therefor. However, a further investigation was made to gain additional conditions for heightening the cell-number ratio.

### Experiment 6

In Experiment 5, the cell-adhering porous body produced using the administration cell concentration of 5 × 10⁷ cells/mL had been stored in the 50-mL preservation medium; in this case, the cell-number ratio had been 90.9%. While the conditions in Experiment 5 were maintained, an inspection was made on additional conditions for improving the survival rate and the cell-number ratio further.

As one of the additional storage conditions, an investigation was made about the shaking speed. The investigation was made about the effect of the shaking speed onto cells of a cell-adhering porous body after a long-term storage thereof. An experiment therefor was made in the same way as in Experiment 5 except that the cell-adhering porous body was prepared, using an administration cell concentration of 5 × 10⁷ cells/mL to adjust the number of administrated living cells to 3 × 10⁶ cells, and the container was shaken at 60 rpm while the cell-adhering porous body was stored.

Under these conditions, the cell-adhering porous body was stored over two weeks, and then the survival rate, the number of living cells, and the cell-number ratio were measured and calculated. The results are shown in Table 7. The individual data are each shown as the average value from three experiment-examples.

**Table 7**

| | | |
|---|---|---|
| Shaking speed | 60 rpm | 15 rpm |
| Administration cell concentration (cells/ml) | 5.0×10⁷ | |
| Survival rate (%) | 86.7 | 72.8 |
| Number of living cells (cells) | 3.66×10⁶ | 3.41×10⁶ |
| Cell-number ratio (compared with that at day 0) | 104.7 | 97.5 |

When the shaking speed was 60 rpm, both of the survival rate and the cell-number ratio were better than when the storage speed was 15 rpm. This result has suggested that when the shaking speed is made large, nutrients contained in the medium are smoothly supplied to the cells in such a porous body.

When the shaking speed was 15 rpm, the flow rate of the preservation medium was substantially unmeasurable with a flow meter or the like (however, the value thereof was not zero). When the shaking speed was 60 rpm, the water surface of the preservation medium went up and down clearly, following the movement of the shaker. The flow rate thereof was from 0.5 to 1.0 cm/s. It can be assumed that as the flow rate of the preservation medium becomes higher, the nutrients contained in the medium are more smoothly supplied to the cells in such a porous body. When the flow rate was each of 15 rpm and 60 rpm, it was verified visually that the porous body was not damaged.

This experiment has suggested that the shaking speed is related to the flow rate of the medium supplied to the cells included in a porous body, that is, the amplitude of the shaking of the shaker, the rotation number thereof, the number of reciprocating movements or the inclination angle thereof, and the period thereof. However, when the shaking speed is made extremely high, it is feared that the porous body is damaged. Thus, the rotation number cannot be made extremely high. This matter has suggested that it is important to shake such a cell-adhering porous body at a high speed so far as the porous body is not damaged.

### <Example 4>

Inspection of Storage Temperature.

An inspection was made as to how much the storage temperature can be varied.

### Experiment 7

The investigation was made about the effect of the storage temperature onto cells of a cell-adhering porous body after a long-term storage thereof in a case where the storage temperature is 37 or 32°C.

Cell suspension containing atelocollagen were prepared in the same way as in the above-mentioned items (1) and (2). Each of the cell suspensions having an administration cell concentration of 5.0 × 10⁷ cells/mL or 1.0 × 10⁸ cells/mL was administered to semi-columnar porous body each made of polylactic acid and having a size of 6 (W) × 5 (L) × 3 (H) so that the number of administrated living cells was adjusted to 3.5 × 10⁶ cells or 7.0 × 10⁶ cells. These were allowed to stand still at 37°C in an incubator for two hours to gelatinize atelocollagen. Then, cell-adhering porous bodies had been prepared. Each of these bodies was put into a single-end sealed cylinder in which 50 mL of medium A0 was contained, and then the cylinder was capped. In this way, the cell-adhering porous bodies were each held in the airtight container. The containers were stored for two weeks while being shaken at an environmental temperature of 37 or 32°C at a shaking speed of 60 rpm, using an ultra-compact temperature-constant shaking incubator (V·BR-36, manufactured by Taitec Co., Ltd.).

After the cell-adhering porous bodies were stored under these conditions, the survival rates, the numbers of living cells, and the cell-number ratios were measured and calculated. The results are shown in Table 8. Data about the case of 5.0 × 10⁷ cells/mL at 37°C are shown as the average value from 10 experiment-examples thereof; and data about the case of the same concentration at 32°C, as that from 12. Data about the case of 1 × 10⁸ cells/mL at 37°C are shown as the average value from three experiment-examples thereof; and data about the case of the same concentration at 32°C, as that from five.

**Table 8**

| | | | | |
|---|---|---|---|---|
| Administration cell concentration (cells/ml) | 5.0×10⁷ | | 1.0×10⁸ | |
| Temperature | 37°C | 32°C | 37°C | 32°C |
| Survival rate (%) | 88.4 | 95.4 | 73.2 | 85.8 |
| Number of living cells (cells) | 3.0×10⁶ | 3.9×10⁶ | 3.6×10⁶ | 5.2×10⁶ |
| Cell-number ratio (compared with that at day 0) | 85.8 | 110.4 | 50.7 | 74.8 |

When the one of the two cell-adhering porous bodies prepared at the administration cell concentration of 5.0 × 10⁷ cells/mL was stored at 32°C, the cell-number ratio was 110.4% and the survival rate was 95.4%. Under this condition, better storage was attained than under the other conditions in Table 8.

In the case of the two cell-adhering porous bodies prepared at the administration cell concentration of 1.0 × 10⁸ cells/mL, better results about both of the cell-number ratio and the survival rate were obtained by the storage at 32°C than by that at 37°C.

In FIG. 5 are shown results obtained by showing, as a graph, the individual cell-number ratio values shown in Table 8. It has been made evident from the results in FIG. 5 that by lowering the storage temperature from 37°C to 32°C, such cell-adhering porous bodies can be stably stored. However, it is understood that in the case where the administration cell concentration is 1 × 10⁸ cells/mL, the survival rate and the cell-number ratio are lower than the case where the concentration is 5.0 × 10⁷ cells/mL, so that the former concentration makes the storability of the cells lower. It has been therefore considered that for the preparing of such cell-adhering porous bodies, it is desired to use the concentration of 5.0 × 10⁷ cells/mL.

### Experiment 8

It has been able to be verified that better storage can be attained at the storage temperature of 32°C than at that of 37°C in Experiment 7. Furthermore, an inspection was made as to how much the storage temperature can be varied in Experiment 8.

A cell suspension containing atelocollagen was prepared in the same way as in the above-mentioned items (1) and (2). The cell suspension having an administration cell concentration of 5.0 × 10⁷ cells/mL was administered to semi-columnar porous bodies each made of polylactic acid and having a size of 6 (W) × 5 (L) × 3 (H) so that the number of administrated living cells was adjusted to 3.5 × 10⁶ cells. These were allowed to stand still at 37°C in an incubator for two hours to gelatinize atelocollagen. Then, cell-adhering porous bodies had been prepared. Each of these bodies was put into a single-end sealed cylinder in which 50 mL of medium A0 was contained, and then the cylinder was capped. In this way, the cell-adhering porous bodies were held in the airtight containers, respectively. The containers were each stored for two weeks while being shaken at an environmental temperature of 30°C, 32°C or 34°C at a shaking speed of 60 rpm, using an ultra-compact temperature-constant shaking incubator (V·BR-36, manufactured by Taitec Co., Ltd.).

After the cell-adhering porous bodies were stored under these conditions, the survival rates, the numbers of living cells, and the cell-number ratios were measured and calculated. The results are shown in Table 9. Data about each of 30°C and 34°C are shown as the average value from three experiment-examples thereof; and data about 32°C, as the average value from 6 experiment-examples.

Table 9 shows results of the respective storages at 30°C, 32°C and 34°C.

**Table 9**

| | | | |
|---|---|---|---|
| Administration cell concentration (cells/ml) | 5.0×10⁷ | | |
| Temperature | 30°C | 32°C | 34°C |
| Survival rate (%) | 97.3 | 97.3 | 95.8 |
| Number of living cells (cells) | 3.26×10⁶ | 3.33×10⁶ | 3.65×10⁶ |
| Cell-number ratio (compared with that at day 0) | 93.2 | 95.1 | 104.2 |

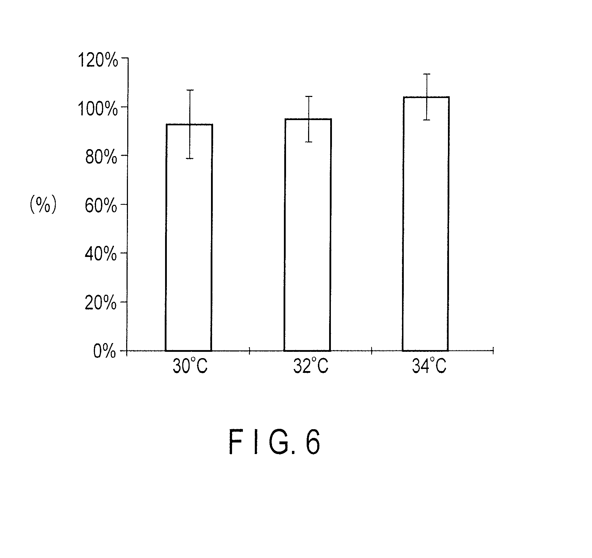

In FIG. 6 are shown results obtained by showing, as a graph, the individual cell-number ratio values shown in Table 9. It has been made evident from the results in FIG. 6 that the cell-adhering porous bodies can be stably stored at each of 30°C, 32°C and 34°C.

At each of 30°C, 32°C and 34°C, the cells kept a satisfactorily survivability. When a safety margin is considered, it has been suggested that the storage at 32°C is preferred.

### <Example 5>

### Investigation on Case where Porous Body Size IS Changed Experiment 9

In Experiments 1, 2, 3 and 4, the porous bodies having the size of 10 (W) × 5 (L) and 3 (H) had been used. In Experiments 5, 6, 7 and 8, the porous bodies having the size of 6 (W) × 5 (L) and 3 (H) had been used. These porous bodies had each been used to prepare the cell-adhering porous body. The investigation had been made on the storage conditions for storing the prepared cell-adhering porous bodies. Furthermore, in Experiment 9, optimal storage conditions were investigated for storing porous bodies having a size of 6 (W) × 50 (L) and 3 (H).

A cell suspension containing atelocollagen was prepared in the same way as in the above-mentioned items (1) and (2). The cell suspension having an administration cell concentration of 5.0 × 10⁷ cells/mL was administered to a semi-columnar porous body made of polylactic acid and having a size of 6 (W) × 50 (L) × 3 (H) so that the number of administrated living cells was adjusted to 3.5 × 10⁷ cells. This was allowed to stand still at 37°C in an incubator for two hours to gelatinize atelocollagen. Then, a cell-adhering porous body A was prepared, and used in Experiment A. In the same way, three cell-adhering porous bodies B, C and D were further prepared, and used in Experiments B to D, respectively.

The resultant cell-adhering porous bodies A, B, C and D were each put into a single-end sealed cylinder containing therein 250 mL of a DMEM medium containing 5 µg/mL of insulin, 0.1 µg/mL of FGFs, and a 10% serum (i.e., medium A5), and then the cylinder was capped. In this way, the cell-adhering porous bodies were each placed in the airtight container. The container was stored for two weeks while being shaken at an environmental temperature of 32°C at a shaking speed of 90 rpm, using an ultra-compact temperature-constant shaking incubator (V·BR-36, manufactured by Taitec Co., Ltd.).

After the cell-adhering porous bodies A, B, C and D were stored under these conditions, the survival rates, the numbers of living cells, and the cell-number ratios were each measured and calculated. The results are shown in Table 10.

**Table 10**

| | Number of living cells after storage for 2 weeks | Cell-number ratio relative to administrated cell amount (3.5×10⁷ cells) | Cell-number ratio relative to cell amount (3.0×10⁷ cells) holdable by porous body * |
|---|---|---|---|
| Experiment A | 3.4×10⁷ cells | 97.1% | 113.3% |
| Experiment B | 3.0×10⁷ cells | 85.7% | 100.0% |
| Experiment C | 2.7×10⁷ cells | 77.1% | 90.0% |
| Experiment D | 3.3×10⁷ cells | 94.3% | 110.0% |
| Average | 3.1×10⁷ cells | 88.6% | 103.3% |

The average cell-number ratio of Experiments A to D in Table 10 is 88.6%.

The number of cells that can be actually held in each of the porous body having the size of 6 (W) × 50 (L) × 3 (H) is 3.0 × 10⁷ cells. Thus, when the measured average cell-number ratio of 88.6% is corrected, considering the number of cells that can be held in the porous body, the corrected average cell-number ratio was 103.3%. This correction was calculated according to the following equation.

Cell-number ratio (%) = Number of living cells collected from the cell-adhering porous body / Number of cells that can be actually kept by the porous body (3.0 × 10⁷ cells) × 100.

These results have made it evident that also in the case of using a porous body having a size of 6 (W) × 50 (L) × 3 (H), the resultant cell-adhering porous body can be satisfactorily stored over a long term.

In Experiments 1 to 8 described above, the porous bodies smaller than those in Experiment 9 had been used. In the case of such a smaller porous body as had been used in Experiments 1 to 8, a loss is hardly generated when a cell suspension is administered into the porous body. By contrast, in the case of such a larger porous body as used in Experiment 8, a loss is generated by the administration thereof. Thus, the number of cells administrated into the porous bodies in Experiment 9 was set to be made large. Apart from this matter, a volume that can be kept by a porous body is a volume obtained by multiplying the volume of the porous body by the porosity thereof, 87%. Consequently, the number of cells that can be actually kept by the porous body is calculated out according to the following calculating equation: 5.0 × 10⁷ cells/mL × 0.59 mL = 3.0 × 10⁷ cells.

The conditions in Experiment 9 were set as follows: The size of the porous bodies to be used in Experiment 9 was 6 (W) × 50 (L) × 3 (H). The porous bodies each had a volume ten times that of the porous bodies used in Experiments 5 to 8, which had the size of 6 (W) × 5 (L) × 3 (H). Thus, the porous bodies to be used in Experiment 9 each had a capacity ten times that of the porous bodies used in Experiments 5 to 8. Accordingly, as the amount of each preservation medium for Experiment 9, the selection of the following amount was conceived: an amount of 500 mL, which is 10 times the amount of 50 mL adopted in Experiments 5 to 8. However, on the basis of the consideration in Experiment 5, the amount of the preservation medium was set to 250 mL, and the concentration of serum contained therein was set to 10%. Under the conditions, the experiment was made. In order to gain a circulation equivalent to the preservation medium-circulation caused in each of the airtight containers in Experiments 5 to 8, the shaking speed was set to 90 rpm.

When the shaking speed was set to 90 rpm, the flow rate of the preservation medium was actually from 1.6 to 2.8 cm/s. It has been assumed that when a cartilage-cell-adhering porous body was stored at such a flow rate, nutrients was sufficiently supplied to the cartilage cells and further waste materials from the cells flowed out to an appropriate degree from the porous body so that the cells were able to be satisfactorily stored. When individual Examples described above were considered, the flow rate "u" of a preservation medium may be set as follows: 0 < u ≤ 2.8 cm/s, preferably "u" of from 1.6 to 2.8 cm/s.

### <Example 6>

Cell-adhering porous bodies were stored under the conditions which proved to be preferable in Experiments 1 to 5.

The cell-adhering porous bodies were prepared by the method described in items (1) to (4) in the above-mentioned item 1, "Process for Preparing Cartilage-Cell-Adhering Porous Body." The administration of their cells into their porous bodies was performed by administrating a cell suspension having an administration cell concentration of 5.0 × 10⁷ cells/mL into the porous bodies, these porous bodies being each a semi-columnar porous body made of polylactic acid and having a size of 6 (W) × 50 (L) × 3 (H) so that the number of administrated cells adjusted to 3.5 × 10⁷ cells.

### Long-term Storage

The cell-adhering porous bodies prepared as described above were stored as follows.

The cell-adhering porous body was rapidly taken out from the laboratory dish after the end of the gelatinization. The cell-adhering porous body was put into a single-end sealed cylinder in which preservation medium A5 was beforehand held, which contained 5 µg/mL of insulin, 0.1 µg/mL of FGFs, a 10% serum, and penicillin/streptomycin. The cylinder was then capped. In this way, the cell-adhering porous body was placed in the air-tight container. In the same way, five porous-body-held air-tight containers were prepared. Each of the preservation medium used in this process was prepared in one of the respective single-end sealed cylinders of the airtight containers before the end of the gelatinization: first, 225 mL of a DMEM was added thereto by means of a 50-mL pipette; next, thereto was added 2.5 mL of 100 units/mL penicillin - 0.1 mg/mL streptomycin by means of a 5-mL pipette; furthermore, thereto was added 250 µL of FGFs having a quantity of 100 µg/mL by means of a 1000µL chip therefor; next, thereto was added 350 µL of NOVOLIN R as insulin by means of a 1000µL chip therefor; thereto was further added 25 ml of serum by means of a 25-mL pipette; and then all of the components were sufficiently mixed with each other to prepare the preservation medium.

The five airtight containers each containing the cell-adhering porous body were incubated at 32°C while being shaken at 90 rpm. After two hours, from one of the five airtight containers, the cell-adhering porous body was taken out, and used for sterile test. After seven days, from one of the four remaining airtight containers, the cell-adhering porous body was taken out, and then subjected to biochemical tests and an mRNA test. After 14 days from the start of the shaking, from one of the three remaining airtight containers, the cell-adhering porous body was taken out to measure the number of cells therein. Results of the tests were then obtained. When these results satisfied standards for delivery, the cell-adhering porous bodies in the state of being held in the two remaining airtight containers were to be delivered for being used for transplantation.

The thus stored cell-adhering porous bodies showed a good cell survival rate and cell-number ratio. The present invention has made it possible to use a cell-adhering porous body prepared by causing cartilage cells to adhere onto a porous body to store the cartilage cells in a satisfactory state over a term for a sterile test prescribed in the Japanese Pharmacopoeia, that is, over 14 days, without exchanging a medium therefor.

In the present examples, cases where the shaking operation was according to the circular motion the diameter of which was 20 mm have been demonstrated. However, the operation is not limited thereto. The diameter of the circular motion may be set into the range of 10 to 50 mm. The same advantageous effects can be attained by supplying such a medium to cells included in a cell-adhering porous body by shaking according to not any circular motion but a reciprocating motion, or by shaking using an inclination using a sliding pedestal.

The shaking operation is set to a circular motion the diameter of which is from 10 to 50 mm; in this case, when it is supposed that the flow rate of such a medium is in proportion to the diameter of the circular motion, the flow rate is in a range from a level of more than 0 to about 7 cm/s. As far as the porous body is not damaged, it is expected that favorable results can be gained by giving a flow rate in this range to the medium. When the shaking operation is made according to a linear motion, the medium is more powerfully hit onto the inner wall of the airtight container than when made according to a circular motion. As a result, it appears that in a moment the medium is partially heightened in flow rate. It is however considered that the flow rate of the medium falls in substantially the same range as a whole.

The components of the basal medium used in the present examples included inorganic salts such as potassium chloride and sodium chloride; amino acids such as L-arginine·HCl, L-cysteine·2HCl, L-histidine·HCl·H₂O, L-isoleucine, L-leucine, L-lysine-HCl, L-methionine, L-phenylalanine, L-threonine, L-tryptophan, L-tyrosine·2Na·2H₂O, and L-valine; vitamins such as choline chloride, folic acid, niacinamide, D-pantothenic acid, pyridoxal·HCl, riboflavin, and thiamine·HCl; saccharides such as glucose. However, not all these components are essential. The same or similar advantageous effects can be produced as far as a medium to be used is a medium for cultivating cells.

## Claims

1. A method for storing a cartilage-cell-adhering porous body in an airtight state over a long term of at shortest 14 days, comprising:
suspending isolated cartilage cells into a 2 to 3% atelocollagen at an administration cell concentration of 1.0 × 10⁷ cells/ml to 1.0 × 10⁸ cells/ml to prepare a cell suspension;
seeding the cell suspension to a biocompatible porous body;
gelatinizing the cell suspension in/on the porous body to yield the cartilage-cell-adhering porous body; and
keeping the cartilage-cell-adhering porous body at a temperature of 26 to 37°C in a medium having an amount of 1 mL or more per 1.0 × 10⁵ cells that are administrated living cells out of the cartilage cells, the medium containing insulin, a fibroblast growth factor, and a 5% or more serum, while the porous body is shaken to set the shaking speed of the medium in the range of 0 cm/s or more and 2.8 cm/s or less.

2. The method according to claim 1, wherein the shaking speed ranges from 1.6 cm/s to 2.8 cm/s.

3. The method according to claim 1, wherein when the amplitude of the shaking is linear, the shaking speed is from 10 mm to 50 mm in at least one direction, and when the amplitude is along a circular direction, the circular direction shaking is attained by shaking at a shaking speed of 15 rpm to 90 rpm along a circle having a diameter of 10 mm to 50 mm.

4. The method according to any one of claims 1 to 3, **characterized in that** the cartilage cells are auricular cartilage cells.

5. The method according to any one of claims 1 to 4, **characterized in that** the concentration of the serum in the medium is 5 volume % or more by volume.

6. The method according to any one of claims 1 to 5, **characterized in that** the concentration of the serum in the medium is 10 volume % or more by volume.

7. The method according to any one of claims 1 to 6, **characterized in that** the medium is a DMEM/F12.

8. The method according to any one of claims 1 to 7, **characterized in that** the temperature for the storage is from 30 to 34°C.
